# EUROPEAN PATENT SPECIFICATION

(11) **EP 3 293 273 B1**
(45) Date of publication and mention of the grant of the patent: **04.11.2020**
(21) Application number: 17190019.4
(22) Date of filing: 08.09.2017
(51) Int. Cl.: C12Q 1/6886

(54) **METHOD FOR ASSISTING JUDGMENT OF PROGNOSIS OF BREAST CANCER, AND APPARATUS FOR ASSISTING JUDGMENT OF PROGNOSIS OF BREAST CANCER**
VERFAHREN ZUR UNTERSTÜTZUNG BEI DER BEURTEILUNG DER PROGNOSE VON BRUSTKREBS UND VORRICHTUNG ZUR UNTERSTÜTZUNG BEI DER BEURTEILUNG DER PROGNOSE VON BRUSTKREBS
PROCÉDÉ D'AIDE À L'ÉVALUATION DU PRONOSTIC DU CANCER DU SEIN ET APPAREIL D'AIDE À L'ÉVALUATION DU PRONOSTIC DU CANCER DU SEIN

(30) Priority: 09.09.2016 JP 2016176302
(43) Date of publication of application: 14.03.2018
(73) Proprietor: National Cancer Center, Tokyo 1040045 (JP); SYSMEX CORPORATION, Kobe-shi Hyogo 651-0073 (JP)
(72) Inventor: Yamada, Tesshi, Tokyo, 1040045 (JP); Yoshida, Masayuki, Tokyo, 1040045 (JP); Masuda, Mari, Tokyo, 1040045 (JP); Sugano, Teppei, Tokyo, 1040045 (JP); Honda, Kazufumi, Tokyo, 1040045 (JP)
(74) Representative: De Clercq & Partners

(56) References cited:
- US-A1- 2012 077 694
- KAZUFUMI HONDA: "The biological role of actinin-4 (ACTN4) in malignant phenotypes of cancer", CELL & BIOSCIENCE, vol. 5, no. 1, 18 August 2015 (2015-08-18) , XP055436397, DOI: 10.1186/s13578-015-0031-0
- SOHEI YAMAMOTO ET AL: "Actinin-4 gene amplification in ovarian cancer: a candidate oncogene associated with poor patient prognosis and tumor chemoresistance", MODERN PATHOLOGY, vol. 22, no. 4, 16 January 2009 (2009-01-16), pages 499-507, XP055119089, ISSN: 0893-3952, DOI: 10.1038/modpathol.2008.234
- ONO MAKIKO ET AL: "Prognostic impact of Ki-67 labeling indices with 3 different cutoff values, histological grade, and nuclear grade in hormone-receptor-positive, HER2-negative, node-negative invasive breast cancers", BREAST CANCER, JAPANESE BREAST CANCER SOCIETY, TOKYO, JP, vol. 22, no. 2, 13 April 2013 (2013-04-13) , pages 141-152, XP035452488, ISSN: 1340-6868, DOI: 10.1007/S12282-013-0464-4 [retrieved on 2013-04-13]

### SEQUENCE LISTING

<110> National Cancer Center Japan SYSMEX CORPORATION
<120> METHOD FOR ASSISTING JUDGMENT OF PROGNOSIS OF BREAST CANCER, AND APPARATUS FOR ASSISTING JUDGMENT OF PROGNOSIS OF BREAST CANCER
<130> SYSM-124-EP
<150> JP 2016-176302
   <151> 2016-09-09
<160> 2
<170> PatentIn version 3.5
<210> 1
   <211> 3893
   <212> DNA
   <213> Homo sapiens
<220>
   <221> CDS
   <222> (60)..(2792)
<400> 1
agaggggcgg gagctgaggc gggagcggac aggctggtgg gcgagcgaga ggcggcgga      59
   atg gtg gac tac cac gcg gcg aac cag tcg tac cag tac ggc ccc agc      107
   agc gcg ggc aat ggc gct ggc ggc ggg ggc agc atg ggc gac tac atg      155
   gcc cag gag gac gac tgg gac cgg gac ctg ctg ctg gac ccg gcc tgg      203
   gag aag cag cag cgc aag acc ttc acg gca tgg tgc aac tcc cac ctg      251
   cgg aag gca ggc aca cag atc gag aac att gat gag gac ttc cga gac      299
   ggg ctc aag ctc atg ctg ctc ctg gag gtc ata tca ggg gag cgg tta      347
   cct aag ccg gag cgg ggg aag atg aga gtg cac aaa atc aac aat gtg      395
   aac aaa gcg ctg gac ttt att gcc agc aaa ggc gtc aag ctg gtc tcc      443
   atc ggg gca gaa gag att gtg gac ggc aac gca aag atg acc ctg gga      491
   atg atc tgg acc atc atc ctt agg ttc gcc atc cag gac atc tcc gtg      539
   gaa gag acc tcg gcc aag gaa ggg ctc ctt ctc tgg tgc cag aga aag      587
   aca gcc ccg tat aag aac gtc aat gtg cag aac ttc cac atc agc tgg      635
   aag gat ggt ctt gcc ttc aat gcc ctg atc cac cgg cac aga cca gag      683
   ctg att gag tat gac aag ctg agg aag gac gac cct gtc acc aac ctg      731
   aac aat gcc ttc gaa gtg gct gag aaa tac ctc gac atc ccc aag atg      779
   ctg gat gca gag gac atc gtg aac acg gcc cgg ccc gac gag aag gcc      827
   ata atg acc tat gtg tcc agc ttc tac cat gcc ttt tca gga gcg cag      875
   aag gct gaa act gcc gcc aac cgg atc tgt aag gtg ctg gct gtc aac      923
   caa gag aac gag cac ctg atg gag gac tac gag aag ctg gcc agc gac      971
   ctc ctg gag tgg atc cgg cgc acc atc ccc tgg ctg gag gac cgt gtg     1019
   ccc caa aag act atc cag gag atg cag cag aag ctg gag gac ttc cgc     1067
   gac tac cgg cgt gtg cac aag ccg ccc aag gtg cag gag aag tgc cag     1115
   ctg gag atc aac ttc aac acg ctg cag acc aag ctg cgc ctc agc aac     1163
   cgg ccc gcc ttc atg ccc tcc gag ggc aag atg gtc tcg gac atc aac     1211
   aat ggc tgg cag cac ttg gag cag gct gag aag ggc tac gag gag tgg      1259
   ctg ctg aat gag atc cgc agg ctg gag cgg ctc gac cac ctg gca gag      1307
   aag ttc cgg cag aag gcc tcc atc cac gag gcc tgg act gac ggg aag      1355
   gaa gcc atg ctg aag cac cgg gac tac gag acg gcc aca cta tcg gac      1403
   atc aaa gcc ctc att cgc aag cac gag gcc ttc gag agc gac ctg gct      1451
   gcg cac cag gac cgc gtg gag cag atc gcc gcc att gcc cag gag ctc      1499
   aac gag ctg gat tac tac gac tcc cac aat gtc aac acc cgg tgc cag      1547
   aag atc tgt gac cag tgg gac gcc ctc ggc tct ctg aca cat agt cgc      1595
   agg gaa gcc ctg gag aaa aca gag aag cag ctg gag gcc atc gac cag      1643
   ctg cac ctg gaa tac gcc aag cgc gcg gcc ccc ttc aac aac tgg atg      1691
   gag agc gcc atg gag gac ctc cag gac atg ttc atc gtc cat acc atc      1739
   gag gag att gag ggc ctg atc tca gcc cat gac cag ttc aag tcc acc      1787
   ctg ccg gac gcc gat agg gag cgc gag gcc atc ctg gcc atc cac aag      1835
   gag gcc cag agg atc gct gag agc aac cac atc aag ctg tcg ggc agc      1883
   aac ccc tac acc acc gtc acc ccg caa atc atc aac tcc aag tgg gag      1931
   aag gtg cag cag ctg gtg cca aaa cgg gac cat gcc ctc ctg gag gag      1979
   cag agc aag cag cag tcc aac gag cac ctg cgc cgc cag ttc gcc agc 2027
   cag gcc aat gtt gtg ggg ccc tgg atc cag acc aag atg gag gag atc 2075
   ggg cgc atc tcc att gag atg aac ggg acc ctg gag gac cag ctg agc 2123
   cac ctg aag cag tat gaa cgc agc atc gtg gac tac aag ccc aac ctg 2171
   gac ctg ctg gag cag cag cac cag ctc atc cag gag gcc ctc atc ttc 2219
   gac aac aag cac acc aac tat acc atg gag cac atc cgc gtg ggc tgg 2267
   gag cag ctg ctc acc acc att gcc cgc acc atc aac gag gtg gag aac 2315
   cag atc ctc acc cgc gac gcc aag ggc atc agc cag gag cag atg cag 2363
   gag ttc cgg gcg tcc ttc aac cac ttc gac aag gat cat ggc ggg gcg 2411
   ctg ggg ccc gag gag ttc aag gcc tgc ctc atc agc ctg ggc tac gac 2459
   gtg gag aac gac cgg cag ggt gag gcc gag ttc aac cgc atc atg agc 2507
   ctg gtc gac ccc aac cat agc ggc ctt gtg acc ttc caa gcc ttc atc 2555
   gac ttc atg tcg cgg gag acc acc gac acg gac acg gct gac cag gtc 2603
   atc gct tcc ttc aag gtc tta gca ggg gac aag aac ttc atc aca gct 2651
   gag gag ctg cgg aga gag ctg ccc ccc gac cag gcc gag tac tgc atc 2699
   gcc cgc atg gcg cca tac cag ggc cct gac gcc gtg ccc ggt gcc ctc 2747
   gac tac aag tcc ttc tcc acg gcc ttg tat ggc gag agc gac ctg         2792
   tgaggcccca gagacctgac ccaacacccc cgacggcctc caggaggggc ctgggcagcc   2852
   ccacagtccc attcctccac tctgtatcta tgcaaagcac tctctgcagt cctccggggt   2912
   gggtgggtgg gcagggaggg gctggggcag gctctctcct ctctctcttt gtgggttggc   2972
   caggaggttc ccccgaccag gttggggaga cttggggcca gcgcttctgg tctggtaaat   3032
   atgtatgatg tgttgtgctt ttttaaccaa ggaggggcca gtggattccc acagcacaac   3092
   cggtcccttc catgccctgg gatgcctcac cacacccagg tctcttcctt tgctctgagg   3152
   tcccttcaag gcctccccaa tccaggccaa agccccatgt gccttgtcca ggaactgcct   3212
   gggccatgcg aggggccagc agagggcgcc accaccacct gacggctggg gacccaccca   3272
   gcccctctcc cctctctgct ccagactcac ttgccattgc caggagatgg ccccaacaag   3332
   caccccgctt ttgcagcaga ggagctgagt tggcagaccg ggcccccctg aaccgcaccc   3392
   catcccacca gccccggcct tgctttgtct ggcctcacgt gtctcagatt ttctaagaac   3452
   caaaaaaaaa aaaggaaaaa aaacacaaaa caacaaaaac caaaaaaaaa aaaaatcaca   3512
   aaaacaaaaa aactataaaa aagaaagaat taaaaacttt cagagaatta ctatttactt   3572
   tattaactta cggatttatt atataaatat atattcacct agcaacatat ctctgccgtc   3632
   tctcctgctc tcataatgaa gacatagccg attctctgcc cgggcccctt gctgatgctc   3692
   ctccgggtct gcgtcgggcg tgggtctctg gggaccctcc agaggtggag gtgggctgat   3752
   ggcctggctg cctggtggtt gatggttttg ctccccctac cttttttttt tgagtttatt   3812
   ctgattgatt ttttttcttg gtttctggat aaaccaccct ctggggacag gataataaaa   3872
   catgtaatat ttttaagaag g                                             3893
<210> 2
   <211> 911
   <212> PRT
   <213> Homo sapiens
<400> 2

## Description

### TECHNICAL FIELD

The present invention relates to a method for assisting the judgment of the prognosis of breast cancer based on a copy number increase of ACTN4 gene and/or the gene amplification, and an apparatus for assisting the judgment of the prognosis of breast cancer.

### BACKGROUND

As a method for predicting the prognosis of breast cancer, a method based on the proportion of Ki67 positive cells is known (for example, ONO M, et al., Breast Cancer. 2015 Mar; 22(2): 141-52). K. Honda, J. Cell. Biol., 1998; 140: 1383 discloses that the intracellular localization of ACTN4 protein in a patient with breast cancer causes the poor prognosis. US2012/077694 A1 (Gray Joe W. et al.) describes a method for prognosing the outcome of breast cancer using further gene amplification markers. Sohei Yamamoto et al., Modern Pathology. 2009 Jan; 22(4): 499-507 describes Actinin-4 gene amplification in ovarian cancer. Actinin-4 is presented as a candidate oncogene associated with poor patient prognosis.

However, in the method that the proportion of Ki67 positive cells is only used as a prognosis predictive factor of breast cancer, the prognosis is not necessarily predicted accurately. Further, the method for predicting the prognosis using ACTN4 gene as the prognosis predictive marker of breast cancer is not known. ACTN4 gene is located at 19q13.1 in the genome. A. KALLIONIEMI, et al. (Proc. Natl. Acad. Sci. USA., Vol. 91, pp. 2156-2160, March 1994.) reports that the amplification of 19q13.1 is detected in one cell line (MDA-157) of breast cancer. However, it reports the phenomenon occurred only in one of the 15 cell lines of breast cancer, and therefore it is not recognized from the paper that the gene amplification of ACTN4 gene is a marker which can be used clinically. Further, it only reports the gene amplification in the cell line of breast cancer. The relationship between the gene amplification and the prognosis is not described at all.

K. Honda, J. Cell. Biol., 1998; 140: 1383. discloses that the change in the intracellular localization of ACTN4 protein causes the poor prognosis. The inventors verified if the amount of the expressed ACTN4 protein was used as the prognosis predictive marker of breast cancer, and found that the amount is less useful. Based on the finding, the inventors developed a method for predicting the prognosis more accurately.

### SUMMARY OF THE INVENTION

The inventors found that the prognosis of breast cancer can be predicted more accurately by the copy number increase of ACTN4 gene and/or the gene amplification of ACTN4 gene as the prognosis predictive factor, and accomplished the invention.

Therefore, provided is a method for assisting the judgment of the prognosis for a patient with breast cancer, the method comprising the steps of: detecting ACTN4 gene contained in a biological sample of the patient; and judging that the prognosis of breast cancer is poor if at least one selected from the group consisting of the copy number increase of ACTN4 gene and the gene amplification of ACTN4 gene is present; wherein the patient is HER2 negative, hormone receptor positive and lymph node metastasis negative.

Also, described is a reagent kit for using the method as described above, comprising one or more reagents capable of measuring the copy number of ACTN4 gene per cell and/or the ratio of the number of ACTN4 gene to that of centromere of chromosome 19. In particular aspects, the kits also comprise instructions for carrying out the methods of the invention.

Further, provided is a judgment apparatus, comprising at least a computer having a processor and a memory, wherein a program is recorded in the memory and executed by the computer, the program comprising the steps of: acquiring from a measurement apparatus at least one information selected from the group consisting of the copy number increase of ACTN4 gene and the gene amplification of ACTN4 gene in a biological sample taken from a patient with breast cancer, and judging the prognosis of breast cancer in the patient based on the acquired information.

The present invention can provide a method capable of predicting the prognosis of breast cancer more accurately. The present invention can also provide an apparatus for predicting the prognosis of breast cancer.

### BRIEF DESCRIPTION OF THE DRAWINGS

Fig. 1 is a figure showing total survival rates of a positive case group and a negative case group for ACTN4 protein expression in patients with pulmonary adenocarcinoma (Phase I-III).
Fig. 2 is a figure showing total survival rates of a positive case group and a negative case group for ACTN4 protein expression in patients with pancreatic cancer (Phase I-IV).
Fig. 3 is a figure showing a survival rate specific for breast cancer (A) and an event-free survival rate (B) of a positive case group and a negative case group for ACTN4 gene number increase in patients with breast cancer being HER2 negative, hormone receptor positive and lymph node metastasis negative.
Fig. 4 is a figure showing an event-free survival rate of a positive case group and a negative case group for ACTN4 protein expression in patients with breast cancer being HER2 negative, hormone receptor positive and lymph node metastasis negative.
Fig. 5 is a figure showing a survival rate specific for breast cancer (A) and an event-free survival rate (B) of a high Ki67-LI (labeling index) group and low Ki67-LI group in patients with breast cancer being HER2 negative, hormone receptor positive and lymph node metastasis negative.
Fig. 6 is a figure showing a relapse-free survival rate of a positive case group and a negative case group for ACTN4 gene in a Luminal A (low Ki67-LI value) judgment group.
Fig. 7 is a figure showing a relapse-free survival rate of a positive case group and a negative case group for ACTN4 gene in a Luminal B (high Ki67-LI value) judgment group.
Fig. 8 is a flowchart for judging whether the prognosis is good or poor in a subject based on Ki67-LI and ACTN4 gene number increase.
Fig. 9 is a schematic figure showing an example of a judgment apparatus for judging the prognosis of breast cancer in a subject.
Fig. 10 is a block diagram showing a configuration of the judgment apparatus in Fig. 9.
Fig. 11 is a block diagram showing a hardware configuration of the judgment apparatus shown in Fig. 9.
Fig. 12 is a flowchart for judging the prognosis in a subject by using the judgment apparatus shown in Fig. 9.

### DETAILED DESCRIPTION OF THE PREFERRED EMBODIMENTS

The method for assisting the judgment of the prognosis in the patient with breast cancer according to this embodiment (hereinafter, also referred simply as "the method") is targeted for a patient with breast cancer being HER2 negative, hormone receptor positive and lymph node metastasis negative. The judgment of HER2 positive or HER2 negative can be performed by any known method. For example, an immunohistochemistry staining (IHC) method for analyzing the overexpression of HER2 protein, or a fluorescence in situ hybridization (FISH) method for searching the amplification of HER2 gene can be used. For example, the judgment by IHC method is performed based on a score obtained by the occupancy ratio of the stained positive cell. For example, the judgment by FISH method can be performed based on HER2 gene number per a cell. Alternatively, the judgment by FISH method can be performed by counting a fluorescence signal of centromere of chromosome 17 (CEP17) and a fluorescence signal of HER2 gene, and calculating a ratio between total numbers of signals. The judgment of HER2 can be performed in accordance with a guideline (ASCO/CAP guideline) in American Society of Clinical Oncology (ASCO) and College of American Pathologists (CAP).

The judgment of positive or negative for a hormone receptor can be performed by any known method. For example, a method for evaluating the occupancy ratio of the positive cell by IHC method, or a method for evaluating a score obtained by the percentage of the positive cell and the staining intensity can be used. One example of the method for evaluating the percentage of the positive cell is J score. If the percentage of the positive cell is 10 % or more, it is judged to be positive. Examples of the method for evaluating a score obtained by the percentage of the positive cell and the staining intensity are Allred score and H score. In Allred score, it is classified into 8 levels based on the percentage of the positive cell and the staining intensity, and if the level is 3 or more, it is judged to be positive. The judgment of the hormone receptor can also be performed in accordance with the ASCO/CAP guideline.

The judgment of positive or negative for lymph node metastasis can be used by any known method including image diagnosis such as palpation, CT examination, MRI examination, mammography, PET examination and ultrasound examination; and pathological examination or gene examination of a lymph node near a breast (e.g., Sentinel lymph node).

The method of the invention comprises a step of detecting ACTN4 gene contained in a biological sample of the patient with breast cancer. More particularly, the method comprises detecting ACTN4 gene number in a biological sample of the patient. Detecting ACTN4 gene is performed in vitro. The sequence of ACTN4 gene and the amino acid sequence coded by the sequence are well known in the art. Examples include the sequences of human-derived ACTN4 as shown in SEQ ID NO: 1 and SEQ ID NO: 2 (Ensembl ID: ENSG00000130402 and ENST00000252699). The sequence of human-derived ACTN4 is also registered in NCBI (National Center for Biotechnology Information) access number: NM_004924 and NP_004915. The genetic locus of ACTN4 gene is also well known. For example, human ACTN4 gene is located on bases 43,830,167-43,913,010 in chromosome 19. For mammals other than human, any similar gene corresponding to human-derived ACTN4 can be used.

The biological sample used in the step of detecting is not particularly limited as long as the gene amplification of ACTN4 gene can be judged in the sample. The sample is preferably a sample prepared from tissues or cells derived from a breast. Examples of the biological sample include tissues or cells of breast cancer taken from a subject by surgery or biopsy. Circulating tumor cells (CTC) which may be present in blood taken from a subject can also be used.

ACTN4 gene can be detected from the biological sample using by any known gene detection method. Examples include southern blotting method, in situ hybridization (ISH) method, comparative genome hybridization (CGH) method, and quantitative PCR method. Among them, in situ hybridization (ISH) method is preferred.

In the method of the invention, whether the gene amplification of ACTN4 gene is present or not is judged based on the result obtained from the step of detecting. Based on the result from the judgment, whether the prognosis in the subject is good or poor is judged. In the step of judging, the prognosis is judged to be poor if at least one of the copy number increase of ACTN4 gene and the gene amplification of ACTN4 gene is present. Whereas, if both the copy number increase of ACTN4 gene and the gene amplification of ACTN4 gene are not present, then the prognosis can be judged to be good.

As used herein, the term "copy number increase of a gene" refers to a phenomenon that the number of a specific gene is increased by the unusual increase of a chromosome in a cell. For example, a plurality of chromosome 19 may be detected in breast cancer cells. By increasing the chromosome itself, the number of ACTN4 gene is also increased.

As used herein, the term "gene amplification" refers to a phenomenon that a part of a chromosome containing a part of or all of the gene is over-amplified. For example, one copy of ACTN4 gene is present originally in chromosome 19. However, two or more copies of ACTN4 gene may be detected in chromosome 19 in breast cancer-derived cells. It is the over-amplification of ACTN4 gene.

As used herein, the term "ACTN4 gene number increase" refers to a phenomenon that the number of ACTN4 gene is increased by the copy number increase of ACTN4 gene and/or the gene amplification of ACTN4 gene.

Whether the copy number increase of ACTN4 gene and/or the gene amplification of ACTN4 gene are present or not can be detected as follows. Firstly, the number of ACTN4 gene per a cell is calculated. Next, the number of a site which is known not to be subject to the over-amplification in chromosome 19 (e.g., centromere) is calculated. When the number of ACTN4 gene is increased with respect to the number of the region, the number of the chromosome is not increased and ACTN4 gene is amplified. Therefore, it can be judged that the gene amplification of ACTN4 gene is present. In other words, the ratio of the number of ACTN4 gene to that of the site is calculated, and whether the gene amplification of ACTN4 gene is present or not can be judged based on the ratio. When both the number of the site and that of ACTN4 gene are observed to be increased at same level, the chromosome itself is increased. Therefore, it can be judged that the copy number increase of ACTN4 gene is present. As used herein, the terms "ratio" and "percentage" have the same meaning of "the value of the ratio". For example, "ratio of A to B" and "ratio between A and B" are "A/B".

In southern blotting method, a genomic DNA is fragmented, separated by electrophoresis, transcribed to a membrane, and hybridized with a specific probe for ACTN4 gene. The relative copy number of ACTN4 gene is obtained by comparing the strength of the hybridization signal from the specific probe with that from a reference probe hybridized with the same genome region in which the copy number increase is not present.

The in situ hybridization (ISH) method is mainly performed by immobilizing tissues or cells to be analyzed, pre-hybridizing the tissues or cells, hybridizing the specific probe to the nucleic acid in the tissues or cells, washing off the unbound specific probe, and detecting the hybridized specific probe. In the fluorescence in situ hybridization (FISH) method, the specific probe is labeled with a fluorescence reporter. The copy number of ACTN4 gene in a cell is obtained by calculating the number of fluorescence in one cell nucleus of the cell.

As the specific probe used for the FISH method, the probe having the nucleotide sequence of 43.81 Mb-44.02 Mb region in human chromosome 19 is preferably used. The copy number of ACTN4 gene in a genome can be measured with high accuracy and sensitivity by using the probe having the nucleotide sequence of the region.

In the comparative genome hybridization (CGH) method, each DNA is extracted from test cells to be analyzed and reference normal cells. Each DNA is labeled with a different colored fluorescent dye. Each labeled DNA is mixed in equal amount to prepare a mixed solution, and the mixed solution is kept in a dark place at 37°C for 2 to 3 days. A competition binding between the test DNA and the reference DNA occurs at all sites in a chromosome. As a result, if the copy number increase of the gene is present in the test DNA, the fluorescence having the color of the fluorescent dye in which the test DNA is labeled is strongly observed at the position of the slide corresponding to the gene. Conversely, if the copy number decrease of the gene is present in the test DNA, the fluorescence having the color of the fluorescent dye in which the reference DNA is labeled is strongly observed at the position of the slide corresponding to the gene.

In the quantitative PCR method, the ACTN4 gene in the genome becomes a template of the amplification reaction. Therefore, the amplification product is obtained corresponding to the copy number of ACTN4 gene.

Among them, in terms of detection accuracy, in situ hybridization is preferred, and the FISH method is more preferred.

In particular embodiments of the methods of the invention, when the copy number of ACTN4 gene per a cell in the biological sample is 5 or more, a copy number increase of ACTN4 gene may be judged to be present.

The ratio of the number of ACTN4 gene to that of centromere of chromosome 19 can be measured by a method such as the FISH method. For example, a modified bacterial artificial chromosome (BAC) probe containing the 43.81 Mb-44.02 Mb region in human chromosome 19 having the ACTN4 (RP11-118P21) genetic locus is used as the specific probe, and a control clone of a nearby centromere in chromosome 19 is used, and then hybridized with the genomic DNA in the biological sample. The ratio of the number of the ACTN4 gene to that of the centromere of chromosome 19 can be obtained by counting the number of the fluorescence signal of the chromosome 19 centromere and that of the ACTN4 gene, and dividing the number of the fluorescence signal of the ACTN4 gene by that of the chromosome 19 centromere. In particular embodiments of the methods of the invention, if the value of the ratio is 2 or more, a gene amplification of the ACTN4 gene can be judged to be present.

In a particular embodiment of the methods of the invention, the proportion of Ki67 positive cells is measured in addition to detecting ACTN4 gene as described above. In the IHC method, immunostaining can be performed by using an antibody which recognizes Ki67 such as MIB-1 antibody. As a method for evaluating the proportion of Ki67 positive cells, a labeling index (LI) can be used. The LI is a proportion of the number of the positive cells with respect to that of cells to be evaluated, which is represented as a percentage. The infiltrating site is preferably used as the site to be evaluated. If a hotspot is observed, the site containing the hotspot is preferably used as the site to be evaluated. The number of the cancer cell to be evaluated is preferably at least 500 and up to 1,000. By calculating the Ki67 LIs of a patient group of which it is known that the prognosis is poor and of a patient group of which it is known that the prognosis is good, the threshold value of the Ki67 LI can be set a value which can be classified into a good prognosis group and a poor prognosis group with the most precision. The threshold value can be set considering a factor such as the sensitivity, the specificity, the hitting ratio of positive case or the hitting ratio of negative case. Regardless of the staining intensity, the positive image of the nucleus can be regarded as positive.

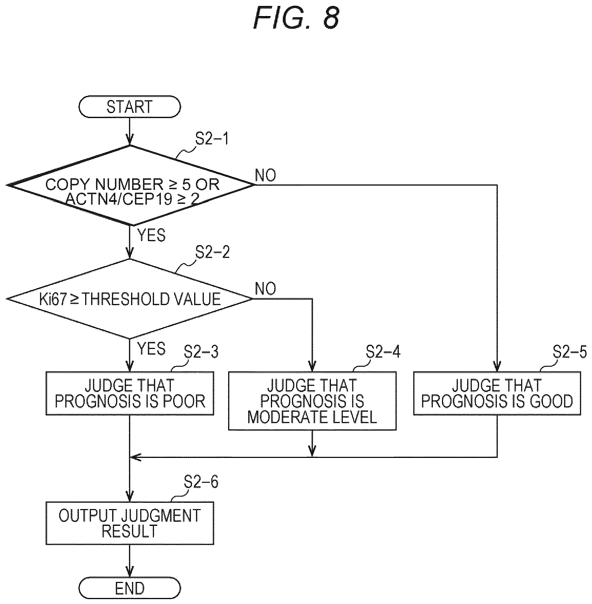

The prognosis of each patient can be judged based on the measurement result of the proportion of Ki67 positive cells (e.g., the Ki67 LI) and the detection result of ACTN4 gene. In particular embodiments, the prognosis of each patient can be classified into three groups, that is, the prognosis is good, the prognosis is poor, and the prognosis is a moderate level which is between good and poor. More specifically, if both the copy number increase of ACTN4 gene and the gene amplification of ACTN4 gene are not detected, then the prognosis can be judged to be good regardless of the Ki67 LI. If the copy number increase of ACTN4 gene and/or the gene amplification of ACTN4 gene is present, and if the Ki67 LI is less than the predetermined threshold value, then the prognosis can be judged to be a moderate level. If the copy number increase of ACTN4 gene and/or the gene amplification of ACTN4 gene is present, and if the Ki67 LI is not less than the predetermined threshold value, then the prognosis can be judged to be poor. Fig. 8 is a flowchart showing a processing flow for predicting the prognosis according to this embodiment. The processing flow will be described later.

In a particular embodiment, the present invention may comprise a method for detecting the ACTN4 gene contained in a biological sample of the patient, characterized in that the copy number increase of ACTN4 gene or the gene amplification of ACTN4 gene is suggestive of poor prognosis of the breast cancer. In the method, the patient is HER2 negative, hormone receptor positive and lymph node metastasis negative. In this embodiment, if the copy number increase of ACTN4 gene and the gene amplification of ACTN4 gene is not found, it may be suggestive of good prognosis. The above-described explanations of the details of the invention is incorporated by reference into this embodiment.

A reagent kit for judging the prognosis of breast cancer (hereinafter, also referred simply as "kit") is within this disclosure. The kit may comprise a reagent capable of measuring the copy number of ACTN4 gene per a cell and/or the ratio of the number of ACTN4 gene to that of centromere of chromosome 19. Preferred example of the reagent includes a specific probe for ACTN4 gene in the genome which can be used for FISH method. The specific probe may preferably contain the nucleotide sequence of 43.81 Mb-44.02 Mb region in human chromosome 19. The kit described herein may contain a centromere probe of human chromosome 19. In a particular aspect, the kit may comprise a reagent capable of measuring the proportion of Ki67 positive cells such as the Ki67 LI. One example of the reagent is an MIB-1 antibody.

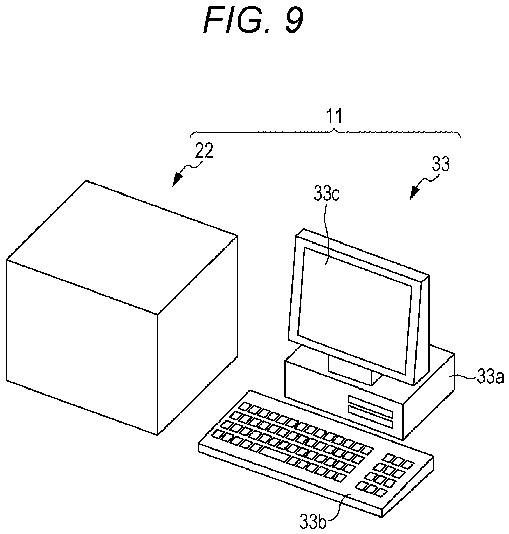

For example, the judgment of the prognosis for a patient with breast cancer can be performed on a judgment apparatus 11 as shown in Fig. 9. Although the judgment apparatus which can be used for the judgment of the prognosis for the patient with breast cancer will be described in more detail with reference to the accompanying Figures, the present invention is not limited to the illustrated embodiments. Fig.9 is a schematic figure of a judgment apparatus for judging the prognosis for the patient with breast cancer according to one embodiment. The judgment apparatus 11 as shown in Fig. 9 comprises a measurement apparatus 22, and a computer system 33 which is connected to the measurement apparatus 22.

In this embodiment, the measurement apparatus 22 may comprise a scanner which detects a signal from the probe connected to ACTN4 gene by in situ hybridization method. In this embodiment, the signal may be an optical information about ACTN4 gene number increase such as a fluorescence signal. When a tissue microarray after the hybridization of the probe is set to the measurement apparatus 22, the measurement apparatus 22 acquires the optical information based on ACTN4 gene connected to the probe in a tissue section. The measurement apparatus 22 transmits the resulting optical information to the computer system 33.

The scanner may be used as long as the scanner can detect the signal based on ACTN4 gene. The signal based on ACTN4 gene is different depending on a labeling material. Therefore, the scanner suitable for detecting the signal generated from the labeling material can be suitably selected depending on the type of the labeling material. For example, if the labeling material is fluorescence, the scanner capable of detecting the fluorescence can be used as the measurement apparatus 22. The optical information detected by the scanner is transmitted to the computer system 33.

The computer system 33 comprises a computer main body 33a, an input device 33b, and a display 33c which displays specimen information, judgment result or the like. The computer system 33 receives the optical information from the measurement apparatus 22. The processor of the computer system 33 executes a program for judging the prognosis for the patient with breast cancer based on the optical information.

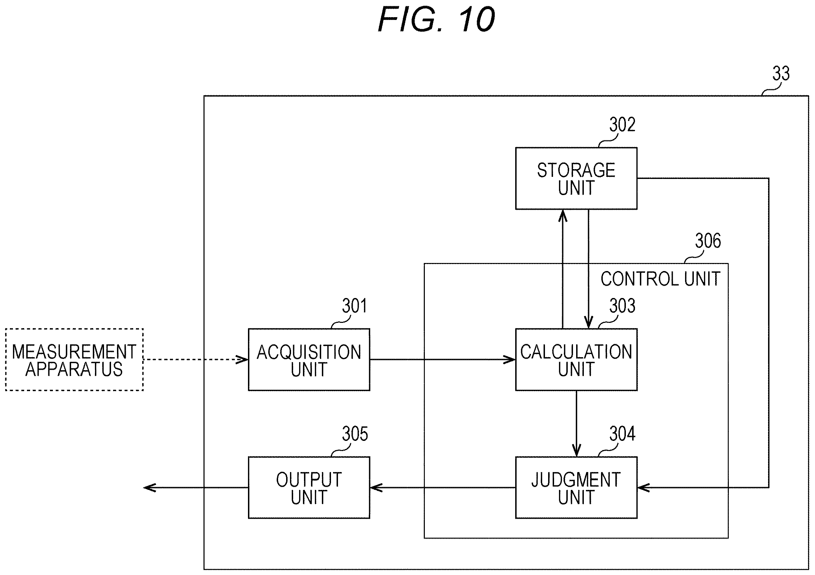

Fig. 10 is a block diagram showing a configuration of the judgment assisting apparatus as shown in Fig. 9. The computer system 33 comprises an acquisition unit 301, a storage unit 302, a calculation unit 303, a judgment unit 304, and an output unit 305, as shown in Fig. 10. The acquisition unit 301 is communicably connected to the measurement apparatus 22 via a network. The calculation unit 303 and the judgment unit 304 constitute a control unit 306.

The acquisition unit 301 acquires the information transmitted from the measurement apparatus 22. The storage unit 302 stores a threshold value for the judgment, an equation for calculating the ratio of the number of ACTN4 gene to that of centromere of chromosome 19, a processing program for judging the prognosis or the like. The calculation unit 303 uses the information acquired in the acquisition unit 301 and the equation, the processing program or the like stored in the storage unit 302 to calculate the number of centromere of chromosome 19 and that of ACTN4 gene, and then calculates the ratio of the number of ACTN4 gene to that of centromere of chromosome 19. The judgment unit 304 judges the prognosis based on the value calculated by the calculation unit 303. The output unit 305 outputs the judgment result by the judgment unit 304 to the display 33c as good prognosis or poor prognosis of breast cancer.

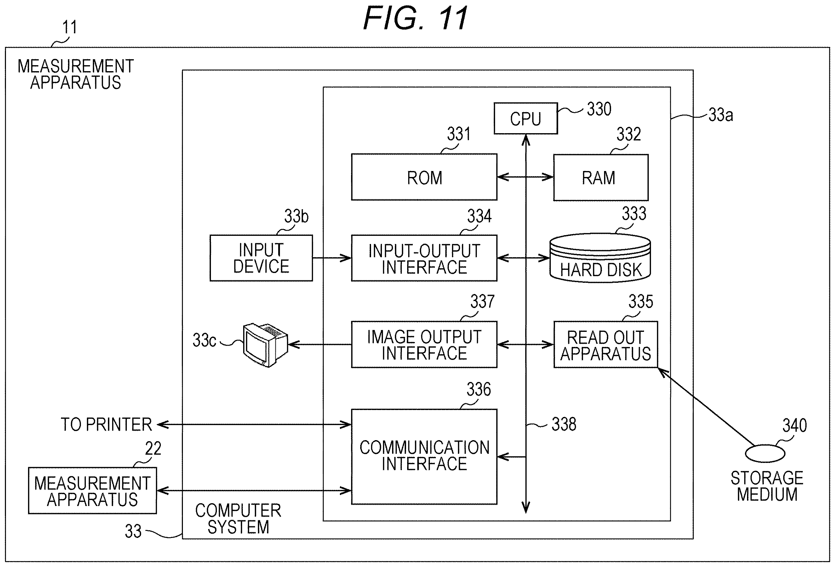

Fig. 11 is a block diagram showing a hardware configuration of the judgment apparatus shown in Fig. 9. As shown in Fig. 11, the computer main body 33a comprises a CPU(central processing unit) 330, a ROM (read only memory) 331, a RAM 332, a hard disk 333, an input-output interface 334, a read-out apparatus 335, a communication interface 336, and an image output interface 337. The CPU 330, the ROM 331, the RAM (random access memory) 332, the hard disk 333, the input-output interface 334, the read-out apparatus 335, the communication interface 336 and the image output interface 337 is data-communicably connected to each other via a bus 338. The CPU 330 can execute a computer program stored in the ROM 331 and a computer program loaded to the RAM 332. The CPU 330 executes an application program and thereby the function blocks as described above are performed.

The computer system acts as a terminal for judgment apparatus for judging that the prognosis for the patient with breast cancer is good or poor.

The ROM 331 is composed of mask ROM, PROM, EPROM, EEPROM or the like. The ROM 331 stores a computer program executed by the CPU 330 and date for using the execution.

The RAM 332 is composed of SRAM, DRAM or the like. The RAM 332 is used for reading the computer program stored in the ROM 331 and the hard disk 333. The ROM 332 is used as a working area for the CPU 330 when the computer program is executed.

An operating system executed in the CPU 330, a computer program such as an application program (a computer program for judging that the prognosis for the patient with breast cancer is good or poor) and data used for executing the computer program are installed in the hard disk 333.

The read-out apparatus 335 is composed of a flexible disk drive, a CD-ROM drive, a DVD-ROM drive or the like. The read-out apparatus 335 can read the computer program or date stored in a portable storage medium 340.

The input-output interface 334 is composed of a serial interface such as USB, IEEE1394, and RS-232C; a parallel interface such as SCSI, IDE, and IEEE1284; and an analog interface such as D/A converter and A/D converter. An input device 33b such as a keyboard or mouse is connected to the input-output interface 334. The operator can input date to the computer main body 33a by using the input device 33b.

An example of the communication interface 336 is Ethernet (registered trademark) interface. The computer system 33 can transmit the printing data for a printer via the communication interface 336.

The image output interface 337 is connected to the display 33c composed of LCD or CRT. The display 33c outputs a video signal depending on the image data obtained from the CPU 330. The display 33c displays the image (picture) following the input video signal.

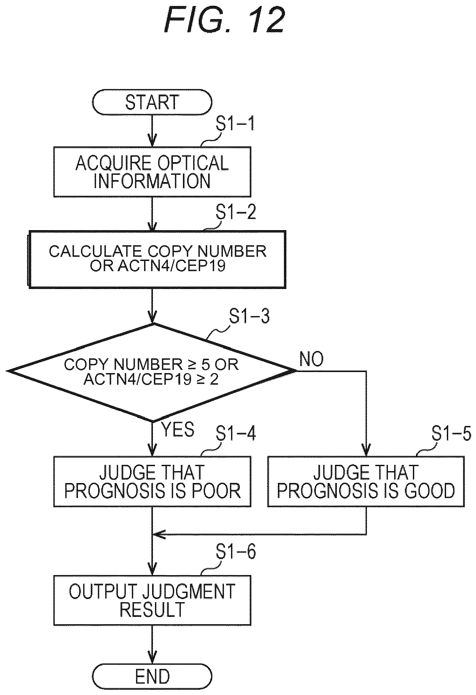

Next, a processing procedure for judging that the prognosis for the patient with breast cancer is good or poor will be described. Fig. 12 is a flowchart for judging the prognosis for the patient with breast cancer by using the judgment apparatus shown in Fig. 9. Although the following example will be described in which the judgment is performed by using the fluorescence information based on ACTN4 gene bound to the probe on the tissue section made by the biological sample of the subject, the disclosure is not limited to the embodiment.

Firstly, in step S1-1, the acquisition unit 301 acquires a fluorescence information from the measurement apparatus 22. Then, in step S1-2, the calculation unit 303 calculates the copy number of ACTN4 gene per a cell and the ratio of the number of ACTN4 gene to that of centromere of chromosome 19 from the fluorescence information which the acquisition unit 301 has acquired, and transmits them to the storage unit 302.

Next, in step S1-3, the calculation unit 303 judges whether the copy number of ACTN4 gene per a cell acquired in the storage unit 302 (represented as "copy number" in Fig. 12) or the ratio of the number of ACTN4 gene to that of centromere of chromosome 19 (represented as "ACTN4/CEP19" in Fig. 12) is greater than the threshold value stored in the storage unit 302 or not. If at least one of the copy number of ACTN4 gene per a cell or the ratio is not less than the threshold value, go to step S1-4. Then, the judgment unit 304 transmits the judgment result showing that the prognosis of the subject is poor to the output unit 305. Whereas, if both the copy number of ACTN4 gene per a cell and the ratio of the number of ACTN4 gene to that of centromere of chromosome 19 are less than the threshold value, go to step S1-5. Then, the judgment unit 304 transmits the judgment result showing that the prognosis of the subject is good to the output unit 305.

After that, in step S1-6, the output unit 305 outputs the judgment result, and the display 33c displays the result or the printer prints the result. It can provide the information for assisting the judgment by a doctor that the prognosis for the patient with breast cancer is good or poor.

The other embodiment of the disclosure is an apparatus that judges the prognosis based on the proportion of Ki67 positive cells and ACTN4 gene. The measurement result and the calculation result about ACTN4 gene are stored in the storage unit 302, as described above. The user inputs Ki67 LI by using the input device 33b. The input Ki67 LI is stored in the storage unit 302. The judgment unit 304 carries out the procedure as shown in Fig. 8 based on the information stored in the storage unit 302.

In step S2-1, the calculation unit 303 judges whether the copy number of ACTN4 gene per a cell acquired in the storage unit 302 (represented as "copy number" in Fig. 8) or the ratio of the number of ACTN4 gene to that of centromere of chromosome 19 (represented as "ACTN4/CEP19" in Fig. 8) is greater than the threshold value stored in the storage unit 302 or not. If at least one of the copy number of ACTN4 gene per a cell or the ratio is not less than the threshold value, go to step S2-2. Then, the calculation unit 303 judges whether Ki67 LI is greater than the threshold value stored in the storage unit 302 or not. If Ki67 LI (represented as "Ki67" in Fig. 8) is greater than the threshold value, go to step S2-3. Then, the judgment unit 304 transmits the judgment result showing that the prognosis of the subject is poor to the output unit 305. Whereas, if Ki67 LI is less than the threshold value, go to step S2-4. Then, the judgment unit 304 transmits the judgment result showing that the prognosis of the subject is a moderate level to the output unit 305.

In the step, if both the copy number of ACTN4 gene per a cell and the ratio of the number of ACTN4 gene to that of centromere of chromosome 19 are less than the threshold value, go to step S2-5. Then, the judgment unit 304 transmits the judgment result showing that the prognosis of the subject is good to the output unit 305.

After that, in step S2-6, the output unit 305 outputs the judgment result, and the display 33c displays the result or the printer prints the result. It can provide the information for assisting the judgment by a doctor that the prognosis for the patient with breast cancer is good, poor, or a moderate level.

### EXAMPLES

Although the disclosure will be now described by Examples, the disclosure is not limited to the Examples in any ways.

### Reference Example 1: Correlation between Phase I pulmonary adenocarcinoma and increased expression of ACTN4 gene

Formalin-fixed, paraffin-embedded resected specimens (total 540 samples) from 372 patients who underwent the surgical resection of Phase I pulmonary adenocarcinoma and 168 patients who underwent the surgical resection of Phase II and III pulmonary adenocarcinoma in National Cancer Center Hospital (Japan) from 1997 to 2009 were used as samples. The expression of ACTN4 protein in the samples was observed by immunohistochemical analysis. The primary antibody used for the immunohistochemical analysis was an anti-human ACTN4 rabbit polyclonal antibody made by National Cancer Center Research Institute (Japan). Immunostaining was performed by ABC method using an avidin-biotin complex.

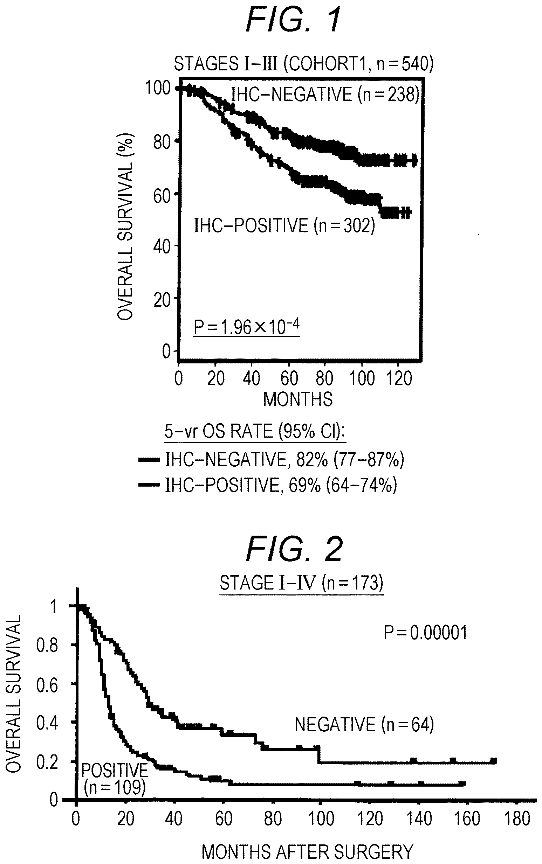

For evaluating the expression of ACTN4 protein, the sample is defined as a positive example when the expression is increased in the cancer moiety in comparison to the vascular endothelium in the same section, and the area having the increased expression accounts for 30% or more of the total area of the tumor. Other samples are defined as negative samples. For two groups, i.e., a positive case group and a negative case group, of the expression of ACTN4 protein, survival curves were made by Kaplan-Meier method, and tested by Log-rank test. The resulting survival curves are shown in Fig. 1. The five-year survival rate was 82% for the negative case group, while the five-year survival rate was 69% for the positive case group. The difference of the survival rate between the negative case group and the positive case group is shown to be increased after 5 years or more from the surgery. Therefore, according to Fig. 1, the prognosis is shown to be poor in the positive case group of the expression of ACTN4 protein in comparison to the negative case group, which is statistically significant.

### Reference Example 2: Correlation between pancreatic cancer and increased expression of ACTN4 gene protein

Formalin-fixed, paraffin-embedded resected specimens from 172 patients who underwent the surgical resection of infiltrating pancreatic ductal carcinoma in National Cancer Center Hospital (Japan) from 1990 to 2003 and had no medical history were used as samples. The expression of ACTN4 protein in the samples was analyzed in the same way as in Reference Example 1.

For evaluating the expression of ACTN4 in the pancreatic cancer cell, the sample is defined as a strong positive when the stainability is clearly higher than the vascular endothelium in the same section. The sample is defined as a weak positive or a negative when the stainability is as same as the vascular endothelium in the same section, or lower than the vascular endothelium in the same section (containing the sample in which the stainability is zero). All samples were classified into the two groups. The classification was performed by three researchers who do not know other clinical data of patients from which the samples were taken. For classified two groups, i.e., a strong positive case group and a weak positive/negative case group of the expression of ACTN4 protein, survival curves were made by Kaplan-Meier method, and tested by Cox-Mantel test. The resulting survival curves are shown in Fig. 2. According to Fig. 2, the prognosis is shown to be poor in the strong positive case group (109 cases) of the expression of ACTN4 protein in comparison to the weak positive/negative case group (64 cases), which is statistically significant.

### Example 1: Correlation between breast cancer and the copy number increase/gene amplification of ACTN4 gene

Formalin-fixed, paraffin-embedded tissue blocks from 369 patients with infiltrating breast cancer being hormone receptor positive, HER2 negative, and lymph node metastasis negative who underwent the surgical resection of primary breast cancer in Tokyo National Cancer Center Hospital (Japan) from 1996 to 2000 were used as samples to construct a tissue microarray.

The tissue blocks were cut into thin pieces having a thickness of 3 to 4 µm to form test samples, and then subjected to fluorescence in situ hybridization (FISH).

FISH probe of bacterial artificial chromosome (BAC) clone having ACTN4 gene and chromosome 19 (reference clone) (Abnova) was used as the probe. The probe was labeled with SpectriumOrange (Abbott Molecular, Des Plaines, IL). FISH was performed with BAC clone DNA. The hybridization was performed at 37°C for 48 hours. The nucleus was counterstained with 4,6-diamidino-2-phenylindole. The number of the fluorescence signal of ACTN4 gene and the number of the fluorescence signal of reference in the nucleus of 20 interphase tumor cells were independently counted by two researchers. If the value obtained by dividing the number of the fluorescence signal of ACTN4 gene by that of reference is 2 or more (gene amplification) or if the average copy number of ACTN4 gene per a cell is 5 or more (copy number increase), the cell is defined as "ACTN4 gene positive". Other cells are defined "ACTN4 gene negative".

The positive/negative classification of ACTN4 gene and various clinical pathological factors were analyzed by univariate analysis and multivariate analysis of Cox regression model. As variables, in addition to ACTN4 gene (positive/negative), age (50 years old or older/under 50 years old), menopause (before/after), size of infiltrating cancer (2 cm or more/ below 2 cm), tissue (infiltrating breast ductal carcinoma/others), histological stage (1-2/3), nuclear grade (1-2/3), lymphatic vessel invasion (positive/negative), Ki67 LI (14 or more/below 14), and ACTN4 protein expression (positive/negative) were used. The results are shown in Table 1.

**[Table 1]**

| | Univariate analysis | | | Multivariate analysis | | |
|---|---|---|---|---|---|---|
| Variables | Hazard ratio | 95% confidence interval | P value | Hazard ratio | 95% confidence interval | P value |
| Age (50 years old or older/under 50 years old) | 0.91 | 0.47 - 1.79 | 0.81 | | | |
| Menopause (before/after) | 0.80 | 0.41 - 1.55 | 0.51 | | | |
| Size of infiltrating cancer (2 cm or more/ below 2 | 1.61 | 0.81-3.19 | 0.23 | | | |
| cm) | | | | | | |
| Tissue (infiltrating breast ductal carcinoma/others) | 0.58 | 0.21 - 1.67 | 0.32 | | | |
| Histological stage (1-2/3) | 2.34 | 1.2 - 4.58 | 0.012 | 1.36 | 0.41 - 4.53 | 0.62 |
| Nuclear grade (1-2/3) | 2.42 | 1.24 - 4.69 | 0.0089 | 1.67 | 0.50 - 5.54 | 0.40 |
| Lymphatic vessel invasion (positive/negative) | 0.68 | 0.33 - 1.39 | 0.29 | | | |
| Ki67 LI (14 or more/below 14) | 1.84 | 0.94 - 3.56 | 0.085 | | | |
| ACTN4 protein expression (positive/negative) | 1.25 | 0.64 - 2.46 | 0.51 | | | |
| ACTN4 gene (positive/negative) | 3.73 | 1.63 - 8.51 | 0.0019 | 3.01 | 1.33 - 7.18 | 0.0088 |

Table 1 shows that ACTN4 gene (positive/negative) is an independent prognosis predictive factor.

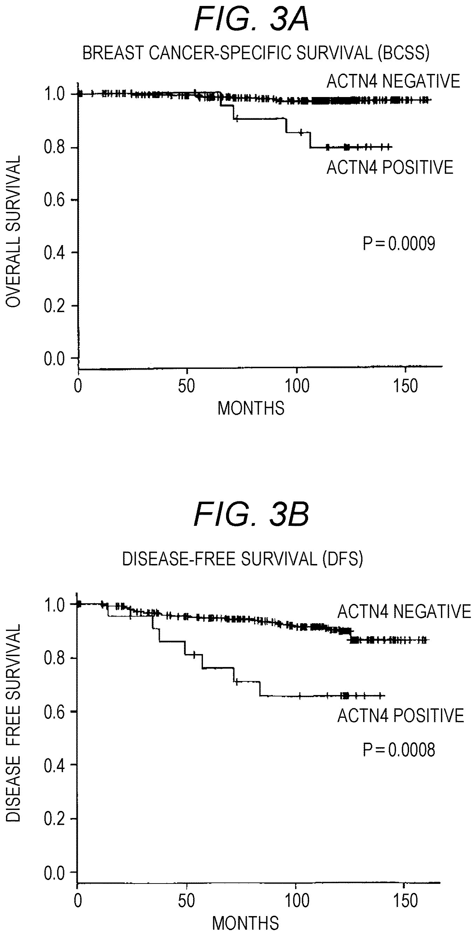

For classified two groups, i.e., a positive case group and a negative case group of ACTN4 gene, survival curves were made by Kaplan-Meier method, and tested by Cox-Mantel test. The disease-specific survival rate (BCSS) was measured during a period from the surgery to death by breast cancer or the final follow-up examination day. The disease-free survival (DFS) was defined as a duration from the surgery to the first detection of new lesion. The resulting survival curves are shown in Fig. 3. According to Fig. 3, the prognosis is shown to be poor in the positive group in comparison to ACTN4 gene negative group for BCSS and DFS, which is statistically significant.

### Comparative Example 1: Correlation between breast cancer and increased expression of ACTN4 gene.

Using the tissue microarray as same as in Example 1, the expression of ACTN4 protein was analyzed by immunohistochemistry analysis (IHC). Immunostaining was performed by using Ventana DABMap detection kit and automatic slide staining (Discovery XT; Ventana Medical System).

For evaluating the expression of ACTN4 protein, the sample is defined as a positive example when the expression is increased in the cancer moiety in comparison to the vascular endothelium in the same section. Other samples are defined as negative samples.

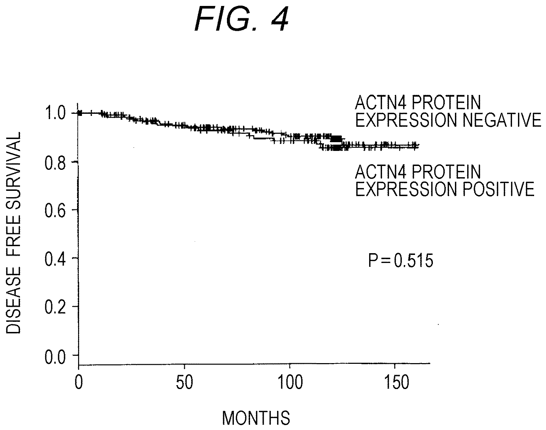

For two groups, i.e., a positive case group and a negative case group of the expression of ACTN4 protein, survival curves were made by Kaplan-Meier method, and tested by Log-rank test. The resulting survival curves are shown in Fig. 4. The result of IHC was evaluated by two independent researchers.

### Comparative Example 2: Correlation between breast cancer and the Ki67 LI

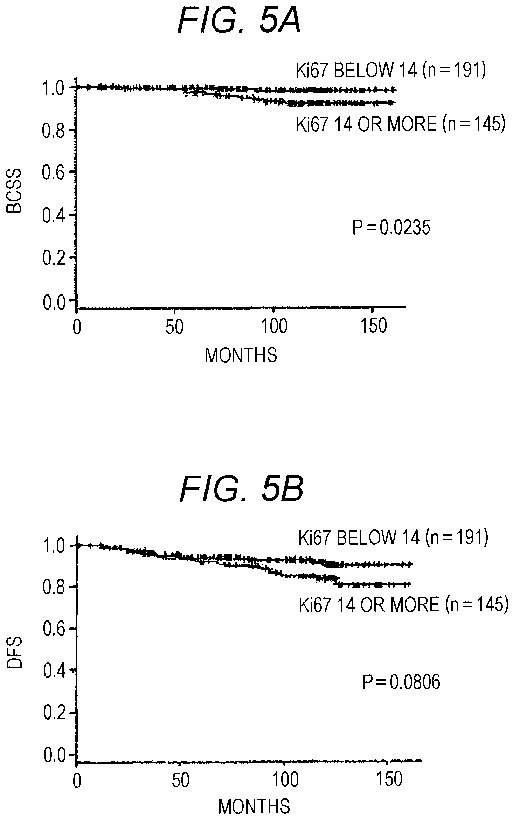

Using the tissue microarray as same as in Example 1, the Ki67 LI was measured by IHC method. MIB-1 (Dako) was used as an anti-Ki67 antibody. IHC was performed by using Autostainer Link 48 (Dako). For the Ki67 LI, two or three regions were selected from area near the central part in the tissue containing the hotspot of a Ki67 positive cell, and a plurality of microphotographs were taken at the different regions. The microphotographs were printed, and the proportion of a Ki67 positive cancer cell showing from moderate to high level of immune response based on 1,000 cancer cells was measured by two independent researchers. The average of the measured value was reported as the Ki67 LI. The group having a Ki67 LI of 14% or more was regarded as Luminal B (high Ki67-LI value) group, and the group having a Ki67 LI of less than 14% was regarded as Luminal A (low Ki67-LI value) group. The survival curves were made by Kaplan-Meier method, and tested by Log-rank test. The resulting survival curves are shown in Fig. 5.

### (Discussion of Reference Example 1, Reference Example 2, Example 1, Comparative Example 1 and Comparative Example 2)

The disclosure in ONO M, et al., Breast Cancer. 2015 Mar;22(2): 141-52 suggests that the possibility that the prognosis can be predicted to be poor by the overexpression of ACTN4 protein. Therefore, the correlation between the expression of ACTN4 protein and the prognosis was confirmed. In lung cancer and pancreatic cancer, the prognosis good group and the prognosis poor group could be distinguished (Reference Example 1 and 2). In contrast, in breast cancer, the prognosis could not be distinguished by the expression of ACTN4 protein (Comparative Example 1). From the result of Comparative Example 1, it is clearly predicted that there is no correlation between ACTN4 gene and the prognosis in breast cancer. However, according to the result of Example 1, the correlation between ACTN4 gene and the prognosis was observed surprisingly, which is statistically significant. According to the results of Example 1 and Comparative Example 2, the prognosis based on ACTN4 gene was predicted with higher accuracy in comparison to Ki 67 LI which is clinically used at present as a poor prognosis marker of breast cancer.

### Example 2

Using the tissue microarray as same as in Example 1, the Ki67 LI was measured by IHC method. MIB-1 (Dako) was used as an anti-Ki67 antibody. IHC was performed by using Autostainer Link 48 (Dako). For the Ki67 LI, two or three regions were selected from an area near the central part in the tissue containing the hotspot of the Ki67 positive cell, and a plurality of microphotographs were taken at the different regions. The microphotographs were printed, and the proportion of Ki67 positive cancer cells showing from moderate to high level of immune response based on 1,000 cancer cells was measured by two independent researchers. The average of the measured value was reported as the Ki67 LI. The group having a Ki67 LI of 14% or more was regarded as Luminal B (high Ki67-LI value) group, and the group having a Ki67 LI of less than 14% was regarded as Luminal A (low Ki67-LI value) group.

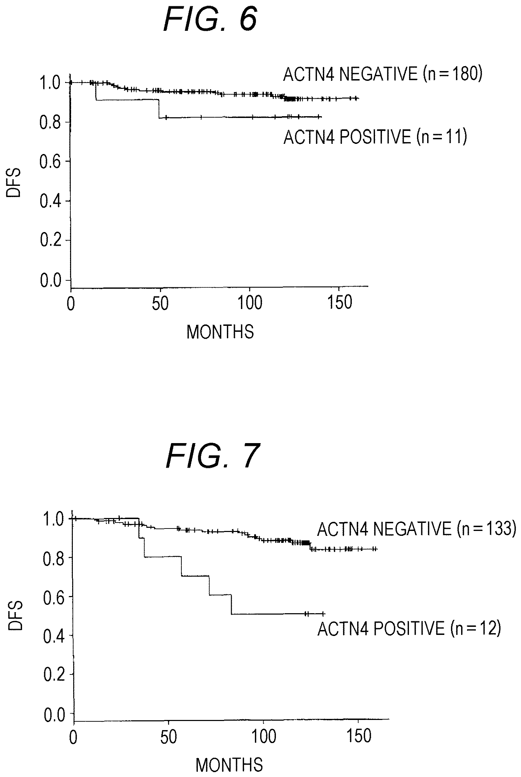

For each group, all samples were classified into the positive group and the negative group of ACTN4 gene by FISH method, as similar as in Example 1. The survival curves were made by Kaplan-Meier method, and tested by Log-rank test. Fig. 6 shows Kaplan-Meier curve of patients in Luminal A (low Ki67-LI value) group. Fig. 7 shows Kaplan-Meier curve of patients in Luminal B (high Ki67-LI value) group.

At present, in groups of patients being hormone receptor positive and HER2 negative, a low Ki67-LI value group is classified in Luminal A, and a high Ki67-LI value group is classified in Luminal B. In Luminal A group, hormone therapy is recommended. In Luminal B group, chemotherapy is recommended in addition to hormone therapy. As shown in Fig. 6, even if the group is a Luminal A group, the prognosis is poor when the patient is ACTN4 gene positive. In contrast, as shown in Fig. 7, even if the group is a Luminal B group, the prognosis is good when the patient is ACTN4 gene negative. Therefore, by judging the prognosis by using ACTN4 gene as the prognosis predictive factor in addition to Ki 67, it suggests that the patient in need of chemotherapy can be selected even if the group is a Luminal A group by Ki 67-LI and that the patient without the need of chemotherapy can be selected even if the group is a Luminal B group by Ki 67-LI.

Figs. 6 and 7 show that the prognosis is good in the negative group of ACTN4 gene regardless of high or low Ki67-LI value (positive or negative). Fig. 7 shows that the prognosis is poor in the group of Ki67-LI positive and ACTN4 gene positive. Fig. 6 shows that the prognosis of the group of Ki67-LI negative and ACTN4 gene positive is between good prognosis group and poor prognosis group. As described above, it suggests that the prognosis can be classified into three groups by using both the proportion of Ki67 positive cells and ACTN4 gene as the prognosis predictive factors.

## Claims

1. A method for assisting the judgment of the prognosis for a patient with breast cancer, the method comprising the steps of:
detecting ACTN4 gene contained in a biological sample of the patient; and
judging that the prognosis of breast cancer is poor if the copy number of ACTN4 gene is 5 or more and/or the gene amplification of ACTN4 gene is present;
wherein the patient is HER2 negative, hormone receptor positive and lymph node metastasis negative.

2. The method according to claim 1, wherein the biological sample is a sample prepared from tissues or cells derived from the breast of the patient.

3. The method according to claim 1 or 2, wherein in the step of judging, if the copy number of ACTN4 gene is less than 5 and the gene amplification of ACTN4 gene is not present, then the prognosis is judged to be good.

4. The method according to any one of claims 1 to 3, wherein in the step of detecting, the number of centromere of chromosome 19 is further detected,
after the step of detecting, the ratio of the number of ACTN4 gene to that of centromere of chromosome 19 is calculated, and
in the step of judging, if the ratio is 2 or more, the gene amplification of ACTN4 gene is judged to be present.

5. The method according to any one of claims 1 to 4, wherein the step of detecting is performed by in situ hybridization.

6. The method according to any one of claims 1 to 5, wherein the subtype of breast cancer is Luminal A or Luminal B.

7. A method for assisting the judgment of the prognosis for a patient with breast cancer, the method comprising the steps of:
detecting ACTN4 gene contained in a biological sample of the patient;
measuring a proportion of Ki67 positive cells in the biological sample of the patient; and
judging the prognosis of breast cancer based on the results from the steps of detecting and measuring;
when the patient is HER2 negative, hormone receptor positive, and lymph node metastasis negative, and
in the step of judging, if the copy number of ACTN4 gene is less than 5 and the gene amplification of ACTN4 gene is not present, the prognosis of breast cancer is judged to be good,
if the copy number of ACTN4 gene is 5 or more or the gene amplification of ACTN4 gene is present, and if the proportion of Ki67 positive cells is less than the predetermined threshold value, then the prognosis of breast cancer is judged to be a moderate level,
if the copy number of ACTN4 gene is 5 or more or the gene amplification of ACTN4 gene is present, and if the proportion of Ki67 positive cells is not less than the predetermined threshold value, then the prognosis of breast cancer is judged to be poor.

8. The method according to claim 7, wherein the proportion of Ki67 positive cells is the Ki67 labeling index.

9. A judgment apparatus, comprising at least a computer having a processor and a memory, wherein a program is recorded in the memory and executed by the computer, the program comprising the steps of:
acquiring from a measurement apparatus at least one information selected from the group consisting of the copy number of ACTN4 gene and the gene amplification of ACTN4 gene in a biological sample taken from a patient with breast cancer, and
judging that the prognosis of breast cancer in the patient is poor if the copy number of ACTN4 gene is 5 or more and/or the gene amplification of ACTN4 gene is present and judging that the prognosis of breast cancer in the patient is good if the copy number of ACTN4 gene is less than 5 and the gene amplification of ACTN4 gene is not present; wherein the patient is HER2 negative, hormone receptor positive and lymph node metastasis negative.

## Patentansprüche

1. Verfahren zur Unterstützung der Beurteilung der Prognose für einen Patienten mit Brustkrebs, wobei das Verfahren die Schritte umfasst:
Nachweisen des ACTN4-Gens in einer biologischen Probe des Patienten; und
Beurteilen, dass die Prognose von Brustkrebs schlecht ist, wenn die Kopienanzahl des ACTN4-Gens 5 oder größer ist und/oder die Genamplifikation des ACTN4-Gens vorliegt;
wobei der Patient HER2-negativ, hormonrezeptorpositiv und lymphknotenmetastasennegativ ist.

2. Verfahren nach Anspruch 1, wobei die biologische Probe eine Probe ist, die aus Geweben oder Zellen hergestellt wurde, die aus der Brust des Patienten abgeleitet wurden.

3. Verfahren nach Anspruch 1 oder 2, wobei in dem Beurteilungsschritt, wenn die Kopienanzahl des ACTN4-Gens geringer ist als 5 und keine Genamplifikation des ACTN4-Gens vorliegt, die Prognose als gut beurteilt wird.

4. Verfahren nach einem der Ansprüche 1 bis 3, wobei in dem Nachweisschritt ferner die Anzahl von Zentromeren des Chromosoms 19 nachgewiesen wird,
nach dem Nachweisschritt das Verhältnis der Anzahl des ACTN4-Gens zu der der Zentromere des Chromosoms 19 errechnet wird, und
in dem Beurteilungsschritt, wenn das Verhältnis 2 oder mehr beträgt, die Genamplifikation des ACTN4-Gens als vorliegend beurteilt wird.

5. Verfahren nach einem der Ansprüche 1 bis 4, wobei der Nachweisschritt durch In-situ-Hybridisierung durchgeführt wird.

6. Verfahren nach einem der Ansprüche 1 bis 5, wobei der Subtyp von Brustkrebs Luminal A oder Luminal B ist.

7. Verfahren zur Unterstützung der Beurteilung der Prognose für einen Patienten mit Brustkrebs, wobei das Verfahren die Schritte umfasst:
Nachweisen des ACTN4-Gens in einer biologischen Probe des Patienten;
Messen eines Anteils an Ki67-positiven Zellen in der biologischen Probe des Patienten; und
Beurteilen der Prognose von Brustkrebs basierend auf den Ergebnissen aus dem Nachweisschritt und dem Messschritt;
wenn der Patient HER2-negativ, hormonrezeptorpositiv und lymphknotenmetastasennegativ ist, und
in dem Beurteilungsschritt, wenn die Kopienanzahl des ACTN4-Gens geringer ist als 5 und keine Genamplifikation des ACTN4-Gens vorliegt, die Prognose als gut beurteilt wird,
wenn die Kopienanzahl des ACTN4-Gens 5 oder größer ist und eine Genamplifikation des ACTN4-Gens vorliegt und wenn der Anteil Ki67-positiver Zellen geringer ist als ein vorbestimmter Schwellenwert, die Prognose von Brustkrebs als in einem moderaten Niveau befindlich beurteilt wird,
wenn die Kopienanzahl des ACTN4-Gens 5 oder größer ist oder die Genamplifikation des ACTN4-Gens vorliegt und wenn der Anteil Ki67-positiver Zellen nicht geringer ist als ein vorbestimmter Schwellenwert, die Prognose von Brustkrebs als schlecht beurteilt wird

8. Verfahren nach Anspruch 7, wobei der Anteil der Ki67-positiven Zellen der Ki-67-Kennzeichnungsindex ist.

9. Beurteilungsvorrichtung, umfassend mindestens einen Computer mit einem Prozessor und einem Speicher, wobei ein Programm in dem Speicher aufgenommen und von dem Computer ausgeführt wird, wobei das Programm die Schritte umfasst:
Erfassen durch eine Messvorrichtung von mindestens einer Information, die aus der Gruppe, bestehend aus der Kopienanzahl des ACTN4-Gens und der Genamplifikation des ACTN4-Gens in einer biologischen Probe ausgewählt ist, die einem Patienten mit Brustkrebs entnommen wurde, und
Beurteilen, dass die Prognose von Brustkrebs bei dem Patienten schlecht ist, wenn die Kopienanzahl des ACTN4-Gens 5 oder größer ist und/oder die Genamplifikation des ACTN4-Gens vorliegt, und
Beurteilen, dass die Prognose von Brustkrebs bei dem Patienten gut ist, wenn die Kopienanzahl des ACTN4-Gens geringer ist als 5 und keine Genamplifikation des ACTN4-Gens vorliegt; wobei der Patient HER2-negativ, hormonrezeptorpositiv und lymphknotenmetastasennegativ ist.

## Revendications

1. Procédé d'aide à l'évaluation du pronostic pour un patient atteint d'un cancer du sein, le procédé comprenant les étapes de :
détection du gène ACTN4 contenu dans un échantillon biologique du patient ; et
évaluation établissant que le pronostic du cancer du sein est mauvais si le nombre de copies du gène ACTN4 est supérieur ou égal à 5 et/ou que l'amplification génique du gène ACTN4 est présente ;
dans lequel le patient est HER2-négatif, positif aux récepteurs hormonaux et négatif aux métastases des ganglions lymphatiques.

2. Procédé selon la revendication 1, dans lequel l'échantillon biologique est un échantillon préparé à partir de tissus ou de cellules issues du sein du patient.

3. Procédé selon la revendication 1 ou 2, dans lequel, dans l'étape d'évaluation, si le nombre de copies du gène ACTN4 est inférieur à 5 et que l'amplification génique du gène ACTN4 n'est pas présente, le pronostic est évalué comme étant bon.

4. Procédé selon l'une quelconque des revendications 1 à 3, dans lequel, dans l'étape de détection, le nombre de centromères du chromosome 19 est en outre détecté,
après l'étape de détection, le rapport du nombre de gènes ACTN4 à celui de centromères du chromosome 19 est calculé, et
dans l'étape d'évaluation, si le rapport est supérieur ou égal à 2, l'amplification génique du gène ACTN4 est évaluée comme étant présente.

5. Procédé selon l'une quelconque des revendications 1 à 4, dans lequel l'étape de détection est réalisée par hybridation in situ.

6. Procédé selon l'une quelconque des revendications 1 à 5, dans lequel le sous-type de cancer du sein est Luminal A ou Luminal B.

7. Procédé d'aide à l'évaluation du pronostic pour un patient atteint d'un cancer du sein, le procédé comprenant les étapes de :
détection du gène ACTN4 contenu dans un échantillon biologique du patient ;
mesure d'une proportion de cellules Ki67-positives dans l'échantillon biologique du patient ; et
évaluation du pronostic du cancer du sein sur la base des résultats issus des étapes de détection et de mesure ;
lorsque le patient est HER2-négatif, positif aux récepteurs hormonaux et négatif aux métastases des ganglions lymphatiques, et
dans l'étape d'évaluation, si le nombre de copies du gène ACTN4 est inférieur à 5 et que l'amplification génique du gène ACTN4 n'est pas présente, le pronostic du cancer du sein est évalué comme étant bon,
si le nombre de copies du gène ACTN4 est supérieur ou égal à 5 ou que l'amplification génique du gène ACTN4 est présente, et si la proportion de cellules Ki67-positives est inférieure à la valeur seuil prédéfinie, alors le pronostic du cancer du sein est évalué comme étant moyen,
si le nombre de copies du gène ACTN4 est supérieur ou égal à 5 ou que l'amplification génique du gène ACTN4 est présente, et si la proportion de cellules Ki67-positives n'est pas inférieure à la valeur seuil prédéfinie, alors le pronostic du cancer du sein est évalué comme étant mauvais.

8. Procédé selon la revendication 7, dans lequel la proportion de cellules Ki67-positives est l'indice de marquage Ki67.

9. Appareil d'évaluation, comprenant au moins un ordinateur possédant un processeur et une mémoire, dans lequel un programme est enregistré dans la mémoire et exécuté par l'ordinateur, le programme comprenant les étapes de :
acquisition à partir d'un appareil de mesure d'au moins une information choisie dans le groupe constitué par le nombre de copies du gène ACTN4 et l'amplification génique du gène ACTN4 dans un échantillon biologique prélevé sur un patient atteint d'un cancer du sein, et
évaluation établissant que le pronostic du cancer du sein chez le patient est mauvais si le nombre de copies du gène ACTN4 est supérieur ou égal à 5 et/ou que l'amplification génique du gène ACTN4 est présente et évaluation que le pronostic du cancer du sein chez le patient est bon si le nombre de copies du gène ACTN4 est inférieur à 5 et que l'amplification génique du gène ACTN4 n'est pas présente ; dans lequel le patient est HER2-négatif, positif aux récepteurs hormonaux et négatif aux métastases des ganglions lymphatiques.
